(19) Europäisches Patentamt
European Patent Office
Office européen des brevets

(11) EP 1 153 062 B1

# (12) FASCICULE DE BREVET EUROPEEN

(45) Date de publication et mention de la délivrance du brevet:
**27.11.2002 Bulletin 2002/48**

(21) Numéro de dépôt: **99958439.4**

(22) Date de dépôt: **16.12.1999**

(51) Int Cl.$^7$: **C08G 77/06**, C12N 11/08, A61K 7/48, C11D 3/37

(86) Numéro de dépôt international:
**PCT/IB99/02019**

(87) Numéro de publication internationale:
**WO 00/037540 (29.06.2000 Gazette 2000/26)**

## (54) POLYMETHYLSILSESQUIOXANE POREUX AYANT DES PROPRIETES ADSORBANTES

PORÖSE POLYMETHYLSILSESQUIOXANE MIT ADSORBIERENDEN EIGENSCHAFTEN

POROUS POLYMETHYLSILSESQUIOXANE WITH ADSORBENT PROPERTIES

(84) Etats contractants désignés:
**AT BE CH CY DE DK ES FI FR GB GR IE IT LI LU MC NL PT SE**

(30) Priorité: **22.12.1998 CH 253398**

(43) Date de publication de la demande:
**14.11.2001 Bulletin 2001/46**

(73) Titulaire: **FIRMENICH SA
1211 Genève 8 (CH)**

(72) Inventeur: **MIMOUN, Hubert
F-01630 Challex (FR)**

(74) Mandataire:
**Salvaterra-Garcia, Maria de Lurdes, Dr.
Firmenich SA,
1, route des Jeunes
1211 Genève 8 (CH)**

(56) Documents cités:
**EP-A- 0 293 795** **EP-A- 0 406 911**

- **PATENT ABSTRACTS OF JAPAN vol. 018, no. 485 (C-1248), 9 septembre 1994 (1994-09-09) & JP 06 157759 A (TOKUYAMA SODA CO LTD), 7 juin 1994 (1994-06-07)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 153 062 B1

## Description

### Domaine technique

La présente demande a trait au domaine des matériaux. Elle concerne, plus particulièrement, un polyméthylsilsesquioxane de nature poreuse et hydrophobe ayant des propriétés remarquables d'adsorption de molécules organiques.

### Technique antérieure

On connaît l'utilisation faite dans l'industrie de matériaux adsorbants tels que, par exemple, les charbons activés, les silices, les alumines ou les zéolites, pour des applications aussi diverses que la purification de l'eau ou de l'air pour la prévention de la pollution, la séparation des liquides ou des gaz par chromatographie, le support de catalyseurs ou l'encapsulation de substances actives, pour ne citer que quelques exemples.

Les propriétés adsorbantes des matériaux dépendent d'un certain nombre de paramètres physiques tels que la densité apparente, la surface spécifique, la taille des particules, le volume poreux, et la distribution poreuse indiquant leur microporosité (<2 nm), leur mésoporosité (entre 2 et 50 nm), ou leur macroporosité (>50 nm). Un support sera également caractérisé par sa stabilité thermique, sa regenerabilité, la réversibilité de ses capacités adsorbantes, son hydrophobicité, son inflammabilité et son absence de toxicité.

L'adsorbant hydrophobe le plus utilisé à présent est le charbon activé. Celui-ci présente cependant l'inconvénient d'être inflammable et de constituer avec l'air des mélanges susceptibles d'exploser. Il est en outre difficile à régénérer et a une capacité d'adsorption limitée.

Une autre classe d'adsorbants couramment utilisés sont les silices. Elles existent dans une grande gamme de surfaces spécifiques et de tailles de particules, et présentent, en conséquence, des propriétés adsorbantes variées.

### Exposé de l'invention

L'objet de la présente demande est un matériau hydrophobe qui appartient à la famille des polyméthylsilsesquioxanes, par la suite abrégé par PMS, qui est thermiquement stable et qui présente la capacité d'adsorber plusieurs fois son propre poids en substance organique.

Plus précisément, la présente demande décrit un polyméthylsilsesquioxane poreux sous forme de poudre contenant des unités

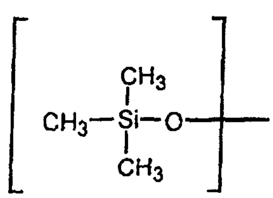

et

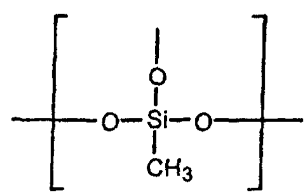

Bouts de chaîne

Modules du materiau

caractérisé par une surface spécifique comprise entre environ 50 et 500 $m^2/g$, un rayon de pores inférieur à 1 nm pour 90% des pores, et l'insolubilité dans l'eau et des solvants organiques.

Dans des modes d'exécution préférés, le polyméthylsilsesquioxane selon l'invention a un volume poreux compris entre 0,1 et 0,8 $cm^3/g$ et/ou un volume de monocouche compris entre environ 10 et 60 $cm^3/g$.

Le cas échéant, le polyméthylsilsesquioxane de l'invention contient aussi des unités

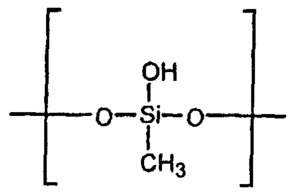

Silanols non polymérisés

Ils peuvent être présents à cause d'une polymérisation incomplète lors de la réaction de préparation décrite ci-dessous, ou d'un traitement qui donne suite à une hydrolyse partielle du PMS.

La présente invention concerne également un procédé pour la préparation dudit polyméthylsilsesquioxane.

Les polyméthylsilsesquioxanes de l'invention sont obtenus par précipitation acide d'une solution aqueuse d'un polyméthylsiliconate, suivie par filtration et séchage. La réaction de préparation des polyméthylsilsesquioxanes de l'invention est illustrée ci-après dans l'équation schématique (1), dans laquelle la formule (I) est représentative d'un polyméthylsiliconate approprié pour une utilisation dans l'invention, et la formule (II) est représentative d'un précipité se formant après l'addition d'un acide approprié par une réaction de condensation du polyméthylsiliconate.

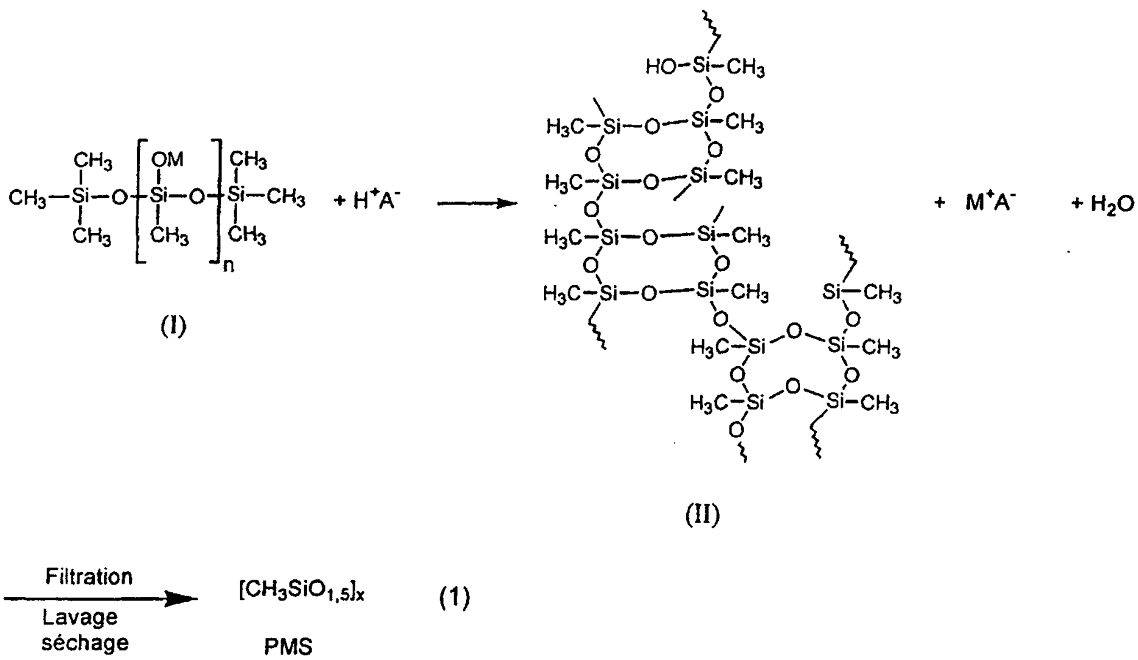


(I) (II)

PMS (1)

M = métal alcalin, alcalino-terreux, $NH_4^+$ ; n = nombre entier de 20 à 100

Le polyméthylsilsesquioxane obtenu par la réaction schématisée ci-dessus et faisant l'objet de l'invention peut être décrit d'une façon approximative par la formule brute $(CH_3SiO_{1,5})_x$. Il n'est toutefois pas possible de préciser l'indicc x, c'est-à-dire de donner le poids moléculaire exact du polyméthylsilsesquioxane, vu que celui-ci est insoluble dans tous les solvants connus, étant donné son poids moléculaire très élevé.

Dans l'équation (1), la lettre M représente un métal alcalin tel que Li, Na, K, un métal alcalino-terreux tel que Mg, Ca, ou un groupe $NH_4^+$, les préférés étant Na et K. L'indice n dans la formule (I) qui est représentatif du nombre des unités répétées $[MOSi(O)CH_3]$ est un nombre entier entre 20 et 100, de préférence entre 30 et 80. Les siliconates caractérisés par la formule (I) sont solubles dans l'eau et peuvent être achetés sur le marché en tant que solutions aqueuses. Nous avons trouvé que l'on obtient de bons résultats lorsqu'on utilise en tant que siliconate le produit connu sous le nom Rhodorsil ® Siliconate 51T, commercialisé par Rhône-Poulenc. Il s'agit d'une solution aqueuse de polyméthylsiliconate de potassium à environ 46% en poids, de pH environ égal à 13, et de densité environ égale à 1,34.

Il est néanmoins aussi possible de préparer les polyméthylsiliconates utilisés dans la synthèse du PMS à partir du polyméthylhydrosiloxane connu sous le nom de PMHS et représenté par la formule (III) suivante :

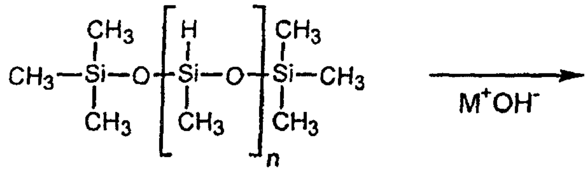


(I) + n/2 $H_2$ (2)

(III)

Dans cette formule (III), n a la même valeur que spécifié auparavant pour les polyméthylsiliconates, à savoir comprise entre environ 20 et 100, de préférence entre environ 30 et 80. En transformant la liaison Si-H en une liaison $Si\text{-}O^+M^-$, M représentant un métal alcalin, alcalino-terreux ou $NH_4^+$, on obtient les polyméthylsiliconates souhaités. Cette transformation peut par exemple se faire par une hydrolyse alcaline en utilisant une base comme le NaOH, le KOH, le $Ca(OH)_2$ ou par l'action d'un alcool suivi d'une hydrolyse alcaline. Il est aussi possible d'utiliser les polyméthylsiliconates qui sont obtenus par la réduction d'un substrat carbonylé en utilisant le PMHS et un catalyseur qui est un sel métallique, de préférence du zinc, suivi d'une hydrolyse alcaline. Un tel procédé fait l'objet de la demande internationale WO 96/12694 au nom de Firmenich SA.

Dans la préparation desdits polyméthylsiliconates, utilisés comme produits de départ, nous avons obtenu les meilleurs résultats, du point de vue des propriétés du PMS obtenu en tant que produit final, lorsque nous avons eu recours à un PMHS ayant une viscosité comprise entre 15 et 50 cSt, de préférence entre 25 et 35 cSt, et une densité comprise entre 0,95 et 1,02.

Comme il ressort de l'équation (1), les polyméthylsiliconates (I) subissent une réaction de condensation lorsqu'ils sont neutralisés à l'aide d'un acide approprié. L'acide est ajouté jusqu'à atteindre un pH d'au moins environ 11, de préférence jusqu'à un pH d'environ 7. L'acide peut même être ajouté jusqu'à un pH d'environ 1. En tant qu'acide, on peut utiliser un acide minéral tel que l'acide sulfurique, l'acide phosphorique, l'acide nitrique, l'acide chlorhydrique, l'acide bromhydrique, l'acide fluorhydrique, l'acide perchlorique ou l'acide fluoroborique. On peut également utiliser un acide organique tel que l'acide acétique, l'acide propionique, l'acide butyrique, l'acide valérique, l'acide 2-éthylhexanoique, l'acide chloroacétique, l'acide dichloroacétique ou l'acide trichloroacétique. On peut également utiliser le gaz carbonique.

On préfère utiliser l'acide acétique, l'acide sulfurique ou l'acide phosphorique, ce dernier étant l'acide le plus utile selon l'invention.

Le précipité qui se forme lors de la neutralisation du polyméthylsiliconate est ensuite filtré ou centrifugé, puis lavé une ou plusieurs fois à l'eau pour enlever les sels alcalins et éventuellement avec un solvant miscible à l'eau tel que, par exemple, le méthanol, l'éthanol, l'isopropanol, ou l'acétone, pour permettre un meilleur séchage du PMS obtenu.

La préparation du PMS s'achève par un séchage sous des conditions permettant d'enlever l'eau et/ou le solvant de lavage. Le séchage peut se faire à des températures comprises entre 80 et 400°C sous pression ambiante ou sous vide.

La température de séchage sera de préférence comprise entre 90° et 150°C, et on utilisera de préférence un vide de 1 à 100 hPa. Le procédé de séchage sera exécuté dans des conditions usuelles, connues de l'homme du métier, par exemple à l'aide d'un sécheur rotatif, cité ici à titre d'exemple non limitatif.

Par le procédé de séchage, on obtiendra ainsi un PMS poreux qui contient moins d'environ 8%, de préférence moins d'environ 5% en poids d'eau. Selon un mode d'exécution encore plus apprécié, on obtiendra un PMS contenant environ 1%, ou moins, en poids d'eau. Comme mentionné auparavant, ce PMS est insoluble dans les solvants organiques de nature hydrophile ou hydrophobe ou dans l'eau.

Le PMS poreux tel qu'obtenu par le procédé décrit auparavant est une poudre fine qui est capable d'adsorber un grand nombre de substances hydrophobes. La densité apparente de cette poudre est comprise entre environ 0,04 et 0,8 g/$cm^3$, selon le mode de préparation. Il s'est avéré que des poudres ayant une densité comprise entre environ 0,08 et 0,4 g/$cm^3$ ont les meilleurs capacités adsorbantes, ces capacités augmentant lorsque la densité diminue. Les données caractéristiques de ces matériaux, qui ont été déterminées par adsorption physique de l'azote (établies sur un appareil de type Sorptomatic 1900 de Carlo Erba) et évaluées selon l'équation de Brunauer-Emmett-Teller (BET), étaient les suivantes :

- surface spécifique comprise entre 50 et 500 $m^2$/g, de préférence comprise entre 75 et 375 $m^2$/g
- 90% des pores ayant un rayon inférieur à 10 angström (1nm), de préférence inférieur à 9 angström (0,9 nm).

Dans un mode d'exécution préféré, le volume poreux du PMS selon l'invention est compris entre 0,1 et 0,8

$cm^3$/g. Il est encore plus apprécié si ce volume est compris entre 0,2 et 0,7 $cm^3$/g.

Dans un autre mode d'exécution préféré, le volume monocouche du PMS selon l'invention est compris entre 10 et 60 $cm^3$/g, et il est encore plus apprécié si ce volume est compris entre 15 et 50 $cm^3$/g.

De plus, la granulométrie moyenne du PMS (qui, bien entendu, varie selon le mode de préparation), est comprise entre 1 et 200 μm, telle que mesurée par diffraction laser en poudre et en suspension dans l'eau en présence d'un tensioactif. Le tensioactif peut, par exemple, être le polyoxyéthylène (20) sorbitane monooléate, en vente sous le nom de Tween® 80 (origine : ICI, Angleterre).

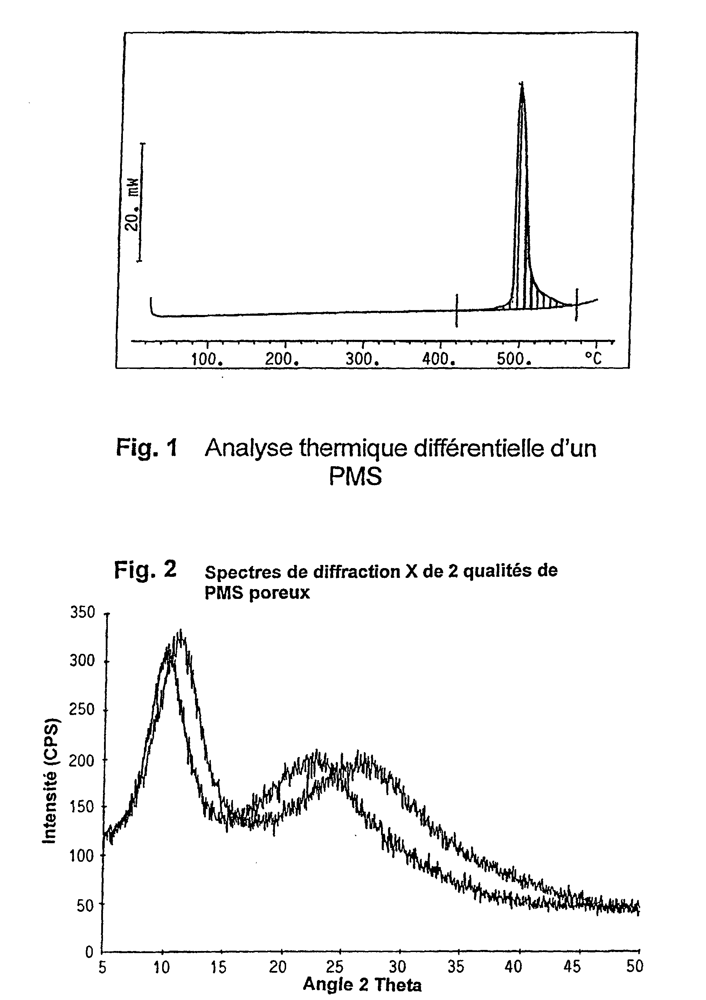

Les mesures thermogravimétriques ont montré que le PMS poreux de l'invention est thermiquement stable jusqu'à une température d'environ 400°C ou légèrement supérieure. Au-delà, des transformations chimiques ont lieu, et à environ 500°C, le PMS perd ses groupes méthyle et se transforme en silice. La figure 1 montre le résultat d'une calorimétrie différentielle sur le PMS de l'invention, exécutée dans les conditions indiquées, laquelle illustre bien le comportement thermique du polyméthylsilsesquioxane de l'invention.

Une autre caractéristique du PMS de l'invention est son caractère essentiellement amorphe. La figure 2 montre les deux bandes d'adsorption (halos) qui sont centrées autour de 10° et de 22° theta obtenues par diffraction aux rayons X. Ces deux bandes susmentionnées sont typiques pour le PMS de l'invention et démontrent qu'il s'agit d'une poudre amorphe présentant un faible degré de cristallinité.

Finalement, le PMS de l'invention se présente sous forme de conglomérats de nanoparticules ayant un caractère poreux, avec des tailles de particule comprises entre environ 1 et 200 μm. On préfère une taille de particules comprise entre environ 2 et 50 μm. Cette taille varie selon les conditions de préparation.

Une caractéristique remarquable du PMS de l'invention est son caractère hydrophobe qui se manifeste par le fait que le PMS flotte sur l'eau et reste entièrement sec. D'un autre côté, grâce à son caractère lipophile, le PMS de l'invention s'associe intimement à toute molécule organique et est capable d'adsorber plus de cinq fois son propre poids en substance organique, comme il sera montré dans les exemples suivants.

Grâce à cette lipophilie, sa surface spécifique et son volume poreux élevé, le PMS de l'invention se prête à de multiples applications, en tant que support solide, d'une grande variété de matières utiles en chimie, parfumerie et/ou cosmétique.

Parmi le grand nombre d'applications envisageables, il s'avère que l'utilisation du PMS en tant que support solide d'enzymes est très avantageuse. Les enzymes fixés sur un support solide tel que, par exemple, la silice, des argiles ou des polyoléfines, sont couramment utilisés dans l'industrie pharmaceutique ou celle des arômes, pour ne citer que deux exemples. L'immobilisation de l'enzyme facilite la séparation de celle-ci du milieu réactionnel et augmente souvent la conversion du substrat par rapport à l'utilisation de la même enzyme non-immobilisée. Or, le PMS de l'invention peut avantageusement remplacer les supports connus dans l'art d'immobilisation d'enzymes.

En tant qu'exemples non limitatifs d'enzymes pouvant être fixées sur le PMS de l'invention, on cite ici les lipases, les péroxydases, les hydrogénases, les lyases, les protéases et les isomérases.

Pour son utilisation en tant que support d'enzymes, le PMS peut être utilisé en tant que tel, ou bien être soumis à un traitement qui facilite la fixation de l'enzyme comme, par exemple, un traitement thermique, acide ou basique, ou une modification de sa surface à l'aide d'organosiloxanes. Les modifications que l'on peut apporter à un support pour arriver à une bonne immobilisation d'enzymes sont connues de l'homme du métier.

Un exemple spécifique d'une réaction dans laquelle le PMS de l'invention trouve une utilisation en tant que support de l'enzyme est la résolution optique d'un mélange d'énantiomères d'un ester ou d'un alcool. Par ce terme, on entend ici, dans le cas d'un ester, l'hydrolyse ou la transestérification énantiosélective, i.e. d'un seul énantiomère, d'un mélange d'énantiomères d'un ester chiral. Dans le cas d'un alcool, ce terme signifie l'estérification énantiosélective d'un mélange d'énantiomères d'un alcool chiral. L'enzyme qui sera en général utilisé dans cette réaction de résolution est une lipase, et on cite ici, en tant qu'exemples de ce type d'enzyme, *Candida antarctica, Pseudomonas fluorescens, Pseudomonas Amano, Humicola lang., Candida cylindracea, Mucor Miehei, Chromabacterium viscosum, Aspergillus niger, Mucor javanicus et Rhisopus arrhizus.*

Le PMS de l'invention peut aussi servir en tant que support de métaux de transition ou de dérivés de ceux-ci, permettant ainsi d'obtenir des catalyseurs hétérogènes ou des catalyseurs homogènes fixés.

Une autre application du PMS de l'invention qu'il convient de citer ici est l'utilisation en tant que réservoir solide pour des substances actives telles que les parfums, arômes, insectifuges ou agents antimicrobiens, par exemple dans le but d'obtenir une action de longue durée de ces substances, grâce à la restitution lente de ces substances actives dans l'air ou l'eau. Par exemple, le PMS de l'invention contenant des composés odorants adsorbés sur sa surface peut être utilisé dans des désodorisants d'air ambiant.

Un autre exemple est l'utilisation du PMS dans des savons, en tant que support pour des parfums qui sont incorporés dans les savons. Nous avons pu constater que l'odeur diffusée par des savons contenant le PMS est préférée à celle du savon ne contenant pas le PMS. La présence du PMS permet une action longue durée du parfum, et souvent le procédé de détérioration du parfum suite aux médias basiques ou acides dans les savons est ralentie.

D'autres applications de ce type incluent les shampoings et autres produits capillaires, tels qu'adoucissants,

laques et shampoings secs, ou encore les produits de type "leave-on", c'est-à-dire les produits de nettoyage et traitement des cheveux qui restent sur ces derniers après application. Nous avons en effet constaté que le PMS était un support très efficace pour les parfums destinés à être incorporés dans ces produits d'hygiène des cheveux, le parfum se libérant de façon contrôlée et plus durable que lorsqu'il était utilisé tel quel, c'est-à-dire non supporté sur le PMS.

Les concentrations dans lesquelles le PMS peut être utilisé dans ces applications peuvent varier dans une gamme de valeurs assez large. On peut citer à titre d'exemple des concentrations de l'ordre de 0,05% à 0,5% en poids, par rapport au poids du produit dans lequel il est incorporé. Cette concentration dépend bien entendu de la quantité de parfum ajoutée au produit et peut être facilement ajustée en fonction de cette dernière.

Le PMS se montre donc très utile comme support du parfum dans toutes les applications de type cosmétique, soins du corps ou des cheveux traditionnellement parfumées, ou encore dans d'autres applications en parfumerie fonctionnelle telles que détergents ou adoucissants textiles par exemple.

On peut aussi l'utiliser dans l'extraction de substances organiques se trouvant en solution ou suspension dans l'eau, comme cela est le cas dans, par exemple, les jus de fruits, les infusions de substances végétales, les vins ou d'autres produits de macération, ou les parfums ou ingrédients parfumants entraînés à la vapeur d'eau. Les propriétés adsorbantes du PMS de l'invention pourront également être utiles dans l'épuration des fumées et des gaz, comme par exemple dans des masques à gaz ou des filtres à cigarettes.

Le PMS a montré une grande utilité dans des savons non parfumés, où il peut avantageusement adsorber les odeurs désagréables diffusées par des bases utilisées dans ces savons.

Le PMS est aussi utile dans la séparation de molécules par chromatographie, grâce à une moindre adsorption de substances polaires par rapport aux substances polaires. Il s'est avéré que le PMS de l'invention est particulièrement adapté à la séparation de molécules par chromatographie dite inverse. Par exemple, nous avons pu constater qu'il était possible de séparer l'extrait de chlorophylle du carotène sur une colonne du PMS de l'invention, par élution avec, au début, un mélange eau/méthanol 50:50, et en augmentant le taux du méthanol jusqu'à arriver au méthanol pur, avec lequel il est possible d'éluer complètement les colorants. Le PMS de l'invention est aussi approprié pour une utilisation dans la chromatographie dite d'exclusion stérique.

Bien entendu, les applications citées auparavant ne constituent pas une liste exhaustive des utilisations potentielles du PMS de l'invention, et beaucoup d'autres applications dans lesquelles les propriétés adsorbantes du PMS seront avantageusement utilisées sont possibles.

L'invention sera maintenant illustrée à l'aide des exemples suivants dans lesquels les abréviations ont le sens usuel dans l'art et les températures sont indiquées en degrés Celsius.

## Exemples

### Exemples 1 à 4

### Préparation du polyméthylsilsesquioxane (PMS)

On dilue 1500 g de polyméthylsiliconate de potassium en solution à 46% dans l'eau avec 6000 g d'eau. Ensuite on neutralise, en maintenant la température inférieure à 30°C, par 486 g d'acide acétique jusqu'à pH = 7. Il se forme aussitôt un précipité blanc qui est filtré, puis lavé deux fois à l'eau. Le précipité est alors séché à 120° dans une étuve sous vide de 10 mbar pendant 12h. On obtient 400 g de PMS poreux sec contenant 1% d'eau résiduelle (analyse Karl Fischer) et une densité = 0,08. Ce PMS (appelé PMS 1) avait une surface spécifique de 191 $m^2/g$, un volume poreux de 0,32 $cm^3/g$ et un volume de la couche monomoléculaire de 44 $cm^3/g$ (mesures effectuées par adsorption physique de l'azote selon la méthode BET). La granulométrie moyenne était de 7 µm (mesure par diffraction laser).

Afin de déterminer sa capacité adsorbante vis-à-vis des substances organiques, on a dosé du limonène contenu dans une burette sur 2 g de PMS 1 contenu dans un drageoir rotatif, jusqu'à ce que celui-ci devienne légèrement collant. On a mesuré alors le volume écoulé qui était de 11 ml, soit 5,5 ml de limonéne adsorbé par g de PMS 1.

En procédant comme décrit ci-dessus pour l'Exemple 1, mais en remplaçant l'acide acétique par de l'acide sulfurique à 10% poids (Ex. 2), l'acide phosphorique à 20% (Ex. 3), ou un courant de gaz carbonique (Ex. 4), on a formé dès pH=10 un précipité blanc que l'on a a filtré, lavé deux fois à l'eau et séché à 120°C sous 10 mbar pendant 12h.

Les propriétés des PMS obtenus après séchage sont indiquées au tableau 1 (PMS 1 à 4). On a constaté que les PMS obtenus avaient des surfaces et des volumes poreux différents et qu'ils adsorbaient dans tous les cas plus de 3ml de limonène par g de PMS.

Tableau 1:

| Comparaison des propriétés adsorbantes des PMS poreux obtenus selon les méthodes des Exemples 1 à 4 | | | | | | |
|---|---|---|---|---|---|---|
| Ex. | Acide | Surface $m^2/g$ | Densité | Limonène adsorbé ml/g | Vol. poreux $cm^3/g$ | Taille des grains µm |
| 1 | $CH_3CO_2H$ | 191 | 0,08 | 5.5 | 0.32 | 7 |
| 2 | $H_2SO_4$ 10% | 275 | 0,086 | 4 | 0.21 | 15 |
| 3 | $H_3PO_4$ 20% | 300 | 0,09 | 4 | 0.3 | |
| 4 | $CO_2$ | 220 | 0,138 | 3.4 | 0.18 | |

Exemples 5 à 9

Extraction de substances organiques de l'eau

On a mis sous agitation à 20°C des solutions ou suspensions contenant 10 g de substance organique dans 1 litre d'eau avec 5 g du PMS obtenu dans l'Exemple 1 (PMS 1) en poudre pendant 10 minutes. On a filtré alors le solide que l'on a pesé, puis on a pesé la phase aqueuse résiduelle que l'on a extraite au diéthyl éther. On détermine ainsi la quantité de substance organique extraite par l'adsorbant dans l'eau.

Comme le montre le tableau 2, le PMS poreux possède la propriété remarquable d'extraire de manière très efficace les substances organiques dans l'eau. Les produits de faible solubilité tels que le toluène (Ex. 5) ou le citral (Ex. 6) sont complètement extraits, alors que les substances très solubles comme le butanol (Ex. 7), la méthyléthylcétone (Ex. 8) ou l'éthylèneglycol diéthyléther (Ex. 9) sont extraits au moins à 70%. Comme le PMS poreux est hydrophobe, il ne contient que de faibles quantités d'eau lorsqu'il est filtré à l'aide d'un solvant organique tel que le méthanol, l'éthanol, l'acétone ou le chlorure de méthylène.

Tableau 2:

| Pourcentage de produit extrait par 5 g de support adsorbant à partir d'une solution contenant 10g de substance organique dans 1 litre d'eau | | |
|---|---|---|
| Exemples | Substance adsorbée | Taux d'extraction |
| 5 | Toluène | 100 % |
| 6 | Citral | 100 % |
| 7 | n-Butanol | 84 % |
| 8 | Méthyléthylcétone | 70 % |
| 9 | Ethylèneglycol diéthyl éther | 75 % |

Exemple 10

Saponification énantiosélective de l'acétate de 2-pentyl-1-cyclopentène-1-yle à l'aide d'une lipase fixée sur un support dérivé du PMS

**A.** Préparation de la lipase fixée

On a préparé une suspension du polyméthylsilsesquioxane de l'invention dans un mélange de propane-2-ol/$H_2O$ 9:1, dans une proportion de 5 ml liquide/g support. La suspension a été agitée pendant 1 h à 50°. On a ensuite refroidi à température ambiante ct continué à agiter pendant 10 h, avant de diluer à l'eau dans une proportion de 1:2. Après 1 h d'agitation de la suspension, celle-ci a été filtrée. Le produit peut être stocké à l'état mouillé.

Pour fixer de la lipase sur ce support prétraité, 1 g d'un adsorbat de *Pseudomonas fluorescens* sur argile (origine : Biocatalysts, Grande-Bretagne) a été suspendu dans 4 ml d'une solution tampon de phosphate à pH7 et 0°. Après agitation pendant 1 h, la suspension a été filtrée et l'extrait dilué 1:1 avec un mélange 1:4 de propan-2-ol / solution tampon de phosphate (0,5 M, pH7, 1 ml) avant d'ajouter le support prétraité comme décrit auparavant (0,5 à 2 g équivalents basé sur poids sec). On a agité la suspension à 50° pendant 15 h avant d'isoler l'enzyme immobilisé par filtration, lavage à l'acétone froide et au pentane. Le produit est séché à l'air à température ambiante.

La lipase immobilisée sur le PMS de l'invention ainsi obtenue peut être utilisée en tant que telle. Elle peut aussi être utilisée dans la préparation d'un biocomposite du type lipase-PMS-silicone. Dans ce but, 10 g de l'adsorbat obtenu comme décrit auparavant ont été dispersés dans un mélange contenant 7 g de polyméthyldisiloxane à terminaisons silanol à haute fonctionnalité (0,9 à 1,2% de fonctionnalité ; origine : ABCR GmbH, Allemagne) et 21 g de polyméthyldisiloxane à terminaisons silanol à basse fonctionnalité (0,1% de fonctionnalité ; origine : ABCR GmbH, Allemagne). Après addition d'un agent de réticulation constitué de 12 g d'un polydiéthylsilicate ayant 45% en poids de solides de silice (origine : ABCR GmbH & Co., Allemagne) et de 0,6 g de 2-éthylhexanoate d'étain (origine : ABCR GmbH & Co., Allemagne), le mélange obtenu a été versé sur l'aluminium. Le temps de gélation à l'air et à température ambiante était d'environ 30 min, le temps de mûrissage d'environ 1 à 2 h. On a obtenu un matériau élastique qui, après broyage, est utilisé dans la réaction d'hydrolyse énantiosélective comme décrit ci-après.

**B.** Réaction de saponification

0,4 g d'un mélange racémique d'acétate de 2-pentyl-1-cyclopentène-1-yle (obtenu comme décrit dans, par exemple, la demande européenne EP-A-841 331 au nom de Firmenich SA) sous forme d'une suspension dans un mélange contenant 0,2 g d'éthanol, 0,2 g d'isopropanol et 0,1 g d'une solution tampon de triéthanolamine à pH 7,5 ont été hydrolysés à l'aide de 50 mg de *Pseudomonas fluorescens* immobilisée, obtenu selon les deux voies de préparation décrites sous A et correspondant à environ 15 g d'enzyme. La température a été maintenue à 25°, pour un temps de réaction de 40 h. On a ensuite procédé à une extraction du mélange à l'aide de méthyl-tert-butyl éther. L'extrait a été analysé par chromatographie en phase gazeuse sur une colonne chirale du type Megadex 5 ayant une longueur de 25 m (100° à 150° à 3°/min). On a obtenu un excès énantiomérique (ee) d'environ 93% et une conversion de près de 100%.

Exemple 11

Utilisation du PMS contenant du parfum adsorbé dans des produits de soins corporels

**A.** Poudre pour bébé

On a mélangé 0,3 g du PMS obtenu dans l'Exemple I avec 0,5 g d'une base parfumante standard pour obtenir une poudre sèche. Celle-ci a été mélangée avec 95,7 g de talc stérilisé (qualité cosmétique) et 3,5 g d'oxyde de zinc micronisé. On a ensuite tamisé le produit ainsi obtenu, afin d'éviter des agglomérats et d'obtenir une poudre pour bébé de la qualité souhaitée.

**B.** Déodorant corporel en poudre

On a mélangé 1,0 g du PMS obtenu dans l'Exemple 1 avec 1,5g d'une base parfumante standard. La poudre sèche qui se forme a été mélangée avec 97,0 g de talc stérilisé et 0,5 g de Triclosan (comme, par exemple, le produit Irgasan ® DP-300 de Ciba SC, Bâle, Suisse). On a ainsi obtenu une poudre qui se prête en tant que déodorant corporel parfumé.

Exemple 12

Préparation d'une composition parfumée pour désodorisant ambiant

On a préparé une composition parfumée destinée à un dispositif désodorisant de l'air ambiant avec les ingrédients suivants :

| | Ingrédients | % en poids |
|---|---|---|
| I. | Dolomite | 5,0 |
| | PMS | 2,0 |
| | Parfum | 13,0 |
| | Tween ® 20 [1)] | 0,2 |
| II. | Eau | 49,4 |

1) monolauréate de polyoxyéthylènesorbitane ; origine : ICI, Grande-Bretagne

(suite)

| | Ingrédients | % en poids |
|---|---|---|
| | Tylose MHB 30.000 2) | 0,4 |
| III. | Plâtre (moulé) | 30,0 |
| | Total | 100,0 |

2) carboxyméthylcellulose ; origine : Hoechst AG, Allemagne

On a bien mélangé les parties I et II séparément et ensuite entre elles. Le mélange ainsi obtenu a été versé dans les moules en plâtre préalablement préparés sous formes décoratives variées.

Exemple 13

Préparation de compositions parfumées pour désodorisants d'air ambiant

On a préparé avec les ingrédients suivants des compositions parfumées pour des gels désodorisants d'air ambiant :

| | | Compositions | |
|---|---|---|---|
| | | A | B |
| | Ingrédients | % en poids | |
| I. | Satiagel K6 1) | 1,3 | 1,3 |
| | Glycérine | 3,0 | 3,0 |
| | Eau | 87,4 | 86,4 |
| | Glydant II 2) | 0,1 | 0,1 |
| | Tylose MHB 30.000 3) | 0,2 | 0,2 |
| II. | Carbonate de magnésium | -- | 2,5 |
| | $SiO_2$ | 3,0 | 1,5 |
| | Parfum | 5,0 | 5,0 |
| | Total | 100,0 | 100,0 |

1) carragénane ; origine : Kelco Int. Ltd., USA
2) conservateur ; origine : Lonza Ind., Suisse
3) voir Exemple 12

On a bien mélangé les parties I et II séparément. Après avoir chauffé la partie I à 75°C, on lui a ajouté la partie II et bien mélangé le tout pour obtenir un mélange homogène.

Les compositions ainsi obtenues ont ensuite été moulées dans des formes diverses, de façon connue en soi, pour fournir des articles désodorisants de l'air ambiant.

Exemple 14

Préparation de savons non parfumés contenant le PMS

On a préparé un savon de base non synthétique à partir des ingrédients suivants :

| Ingrédients | % en poids |
|---|---|
| Sel de sodium d'acide gras de suif ou d'un autre acide gras naturel * | 88,95 |
| Eau | 10,00 |
| Chlorure de sodium | 0,50 |

* palme, stéarine de palme, olive, etc

(suite)

| Ingrédients | % en poids |
|---|---|
| Glycérol | 0,50 |
| Alkali libre | 0,05 (ou acides gras libres 0,5%-1,0%) |
| Total | 100,00 |

On a ensuite préparé des savons sans aucune adjonction et avec l'addition de 1,5% en poids du PMS avec cette base présentant une odeur fort désagréable. Après la préparation, un panel constitué d'évaluateurs n'a pas constaté de différence entre ces deux savons. Les savons ont ensuite été stockés à 40° pendant 30 jours. Ensuite, le panel a largement préféré le savon contenant le PMS, à cause d'un taux d'odeur réduit. Le résultat a été confirmé par le même panel qui a encore évalué l'odeur de ces savons après un autre stockage pendant 2 mois à température ambiante. Toutes les évaluations ont été effectuées à l'aveugle.

Exemple 15

Préparation d'un après-shampoing de type "leave-on"

On a préparé un après-shampoing de type "leave on", c'est-à-dire destiné à ne pas être rincé, avec les ingrédients suivants :

| | Ingrédients | % en poids |
|---|---|---|
| A) | Phytantriol®1) | 0,10 |
| | Renex ® 690 2) | 0,20 |
| | Propylène glycol | 1,00 |
| | D-Panthénol 1) | 0,20 |
| | Eau déionisée | 94,85 |
| | Ethoquad ® O/12 3) | 0,70 |
| | Crosilk liquid 4) | 0,05 |
| | Mackpro NSP 5) | 0,10 |
| | Hydrochlorure d'arginine | 0,10 |
| | Kathon ® CG 6) | 0,05 |
| | Glydant ® 7) | 0,20 |
| | Germall II 8) | 0,20 |
| | Sodium phosphate, tribasique (12H20) | 0,25 |
| | Acide phosphorique (solution aqueuse à 42%) | 0,40 |
| | Dow Corning 929 émulsion cationique 9) | 1,00 |
| B) | PMS 10) . | 0,30 |
| | Parfum 10) | 0,30 |
| | Total | 100,00 |

1) origine : Hoffmann-La Roche
2) Nonoxynol-10 ; origine : ICI Surfactants
3) Alcool isopropylique (et) PEG-2 oleammonium chloride ; origine : Akzo Nobel
4) Poudre de soie ; origine : Croda
5) Quarternium-79 soie hydrolysée ; origine : McIntyre
6) Méthylchloroisothiazoline et méthylisothiazoline; origine : Rohm & Haas
7) DMDM Hydantoine ; origine : Lonza
8) Diazolidinyl Urea, origine : ISP
9) Amodimethicone (et) Nonoxynol-10 (et) Tallowtrimonium Chlorure ; origine : Dow Corning
10) origine : Firmenich SA

On a bien mélangé le Phytanthiol ® avec le Renex ® 690 et ajouté les autres ingrédients de la partie A. Ensuite, on a bien mélangé le PMS de l'invention avec le parfum et laissé pendant la nuit afin d'avoir une absorprion complète du parfum. La partie B ainsi préparée a été ajoutée à la partie A et le tout a été bien homogénéisé. On a

ainsi obtenu un après-shampoing à être agité avant usage et destiné à être appliqué aux cheveux mouillés, sans rinçage postérieur.

Alternativement, on peut également ajouter encore à ce produit un épaississant afin d'empêcher une séparation des particules de PMS parfumé.

Si nécessaire, on ajuste le pH à 4-4,5 à l'aide d'acide phosphorique.

Lorsque ce produit a été appliqué sur les cheveux et comparé, dans un test à l'aveugle, avec un produit similaire mais contenant le parfum sans être supporté sur le PMS, on a constaté que les cheveux traités avec le premier dégageaient l'odeur du parfum pendant beaucoup plus longtemps que lors de l'utilisation de l'après-shampoing parfumé de façon classique.

Exemple 16

Préparation de shampoings

On a préparé un shampoing liquide à l'aide des ingrédients suivants :

| Ingrédients | % en poids |
|---|---|
| Eau déionisée | 64,38 |
| Kathon ® 1) CG | 0,10 |
| Comperlan ®2) KD | 1,50 |
| Texapon ®3) NSO IS | 32,00 |
| Acide citrique | 0,02 |
| Parfum 4) | 0,50 |
| Chlorure de sodium | 1,50 |
| Total | 100,00 |

1) voir Exemple 15
2) cocamide DEA ; origine : Henkel
3) sulfate de sodium laureth ; origine : Henkel
4) origine : Firmenich SA

Les ingrédients ont été bien mélangés pour obtenir une base de shampoing homogène à partir de laquelle on a préparé les compositions suivantes :

| Composition | Ingrédients |
|---|---|
| A | Base + 0,5% en poids de PMS |
| B | Base + 0,5% en poids de Luviquat Hold 5) |
| C | Base + 0,5% de Luviquat Hold 5) + 0,5% PMS |
| D | Base + 0,5% de Jaguar Exel 6) |
| E | Base + 0,5% de Jaguar Exel 6)+ 0,5% PMS |

5) polymère cationique ; origine : BASF
6) polymère cationique : origine : Rhodia

Dans la préparation des compositions C et E le parfum a d'abord été mélangé au PMS comme décrit à l'exemple précédent avant d'intégrer ce mélange dans la base.

Les compositions A à E ont été comparées à l'aveugle par un panel d'évaluateurs auxquels on a demandé d'attribuer une valeur, dans une échelle croissante de 1 à 10, correspondant à l'intensité de l'odeur perçue sur cheveux humides et secs.

Les résultats de ces évaluations sont résumés dans le tableau suivant :

| Composition | Intensité de l'odeur |
|---|---|
| BASE | 3 |
| A | 6 |
| B | 5 |
| C | 6 |

(suite)

| Composition | Intensité de l'odeur |
|---|---|
| D | 6 |
| E | 8 |

Il ressort clairement de ce tableau que lorsque le parfum est ajouté au shampoing sur support de PMS de l'invention, ce dernier améliore nettement le dépôt du parfum sur les cheveux. Par ailleurs, il a été constaté que les cheveux restaient parfumés plus longtemps que lorsque l'on ajoutait le parfum tel quel à la base de shampoing.

Exemple 17

Préparation d'un après-shampoing à rincer

On a préparé un après-shampoing à rincer à l'aide des ingrédients suivants :

| | Ingrédients | % en poids |
|---|---|---|
| A) | Eau déionisée | 91,60 |
| | Solution aqueuse à 20% de chlorhexidine digluconate 1) | 0,25 |
| | Nipagin ® MNa 2) | 0,10 |
| | Nicotinamide 3)98% | 0,05 |
| | Genamin ® KSL 4) | 3,00 |
| | Acide lactique L(+) 85-90% | 0,30 |
| | D-Panthénol 5) | 0,30 |
| | Dow Corning Q2-5200 6) | 0,20 |
| | Alcool éthylique | 1,30 |
| | Lanoline Ultra 7) | 0,20 |
| B) | Jaguar C-162 8) | 1,00 |
| | Natrosol 250 H 9) | 0,70 |
| C) | PMS 10) | 0,50 |
| | Parfum 10) | 0,50 |
| | Total | 100,00 |

1) origine : ICN Biochemicals
2) Méthylparaben de sodium ; origine : NIPA
3) Niacinamide ; origine : Acros Organics
4) PEG-5 Stéaryl Ammonium Lactate ; origine : Clariant
5) origine : Hoffmann-La Roche
6) Laurylméthicone Copolyol ; origine : Dow Corning
7) origine : Westbrook Lanolin
8) Hydroxypropyl Guar Hydroxypropyltrimonium Chlorure ; origine : Rhône-Poulenc
9) Hydroxyéthylcellulose ; origine : Hercules
10) origine : Firmenich SA

La partie A a été chauffée à environ 60°C jusqu'à solution complète de l'alcool cétylique. On a ensuite ajouté la partie B qui avait été mélangée au préalable. On a ensuite homogénéisé jusqu'à ce que la crème ainsi formée ait refroidi à température ambiante.

Le PMS a été bien mélangé au parfum et on a laissé le mélange reposer pendant la nuit pour une bonne absorption. On a ensuite ajouté ce PMS parfumé à la crème préparée auparavant pour obtenir l'après-shampoing désiré. Le pH de ce dernier peut être ajusté à environ 4 si nécessaire, à l'aide d'acide lactique.

**Revendications**

1. Polyméthylsilsesquioxane poreux sous forme de poudre contenant des unités

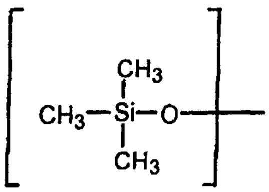

et

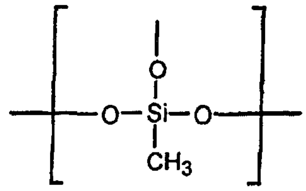

Bouts de chaîne

Modules du materiau

**caractérisé par** une surface spécifique comprise entre environ 50 et 500 $m^2$/g, un rayon de pores inférieur à 1 nm pour 90% des pores, et l'insolubilité dans l'eau et des solvants organiques.

2. Polyméthylsilsesquioxane selon la revendication 1, **caractérisé par** un volume poreux compris entre environ 0,1 et 0,8 $cm^3$/g.

3. Polyméthylsilsesquioxane selon la revendication 1 ou 2, **caractérisé par** un volume de monocouche compris entre environ 10 et 60 $cm^3$/g.

4. Polyméthylsilsesquioxane selon l'une des revendications 1 à 3, **caractérisé par** deux bandes d'adsorption autour de 10° et de 22° obtenues par diffraction aux rayons X.

5. Polyméthylsilsesquioxane selon l'une des revendications 1 à 4, **caractérisé par** une granulométrie moyenne comprise entre environ 1 et 200 µm.

6. Polyméthylsilsesquioxane selon l'une des revendications 1 à 5, **caractérisé par** une stabilité thermique jusqu'à environ 400°C et une température de décomposition d'environ 500°C, telles que déterminées par calorimétrie différentielle et par thermogravimétrie.

7. Polyméthylsilsesquioxane selon l'une des revendications 1 à 6, **caractérisé par** une densité de poudre comprise entre environ 0,04 et 0,8 g/$cm^3$.

8. Polyméthylsilsesquioxane selon l'une des revendications 1 à 7, **caractérisé par le fait qu'**il s'agit d'un conglomérat de nanoparticules poreuses ayant une taille de particules comprise entre environ 1 et 200 µm.

9. Polyméthylsilsesquioxane selon l'une des revendications 1 à 8, **caractérisé en ce qu'**il contient en plus des unités

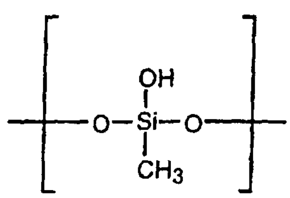

Silanols non polymérisés

10. Polyméthylsilsesquioxane selon l'une des revendications 1 à 9, **caractérisé par** une surface spécifique comprise entre environ 75 et 375 $m^2$/g, un volume poreux compris entre environ 0,2 et 0,7 $cm^3$/g, un volume de monocouche compris entre environ 15 et 50 $cm^3$/g et un rayon de pores inférieur à 0,9 nm pour 90% des pores.

**11.** Procédé de préparation d'un polyméthylsilsesquioxane poreux sous forme de poudre, ce procédé comprenant la précipitation à l'aide d'un acide d'une solution aqueuse contenant un polyméthylsiliconate de formule générale

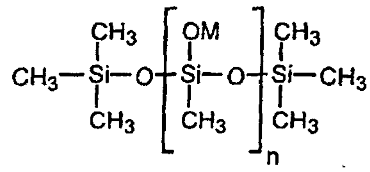

(I)

dans laquelle n est un nombre entier compris entre environ 20 et 100 et M est un métal alcalin ou alcalino-terreux ou $NH_4^+$.

**12.** Procédé selon la revendication 11, **caractérisé en ce que** l'acide est ajouté à la solution jusqu'à atteindre un pH d'au moins environ 11.

**13.** Procédé selon la revendication 11, **caractérisé en ce que** l'acide est ajouté à la solution jusqu'à atteindre un pH d'au moins environ 7.

**14.** Procédé selon l'une des revendications 11 à 13, **caractérisé en ce que** M est le sodium ou le potassium.

**15.** Procédé selon l'une des revendications 11 à 14, **caractérisé en ce que** l'acide utilisé est l'acide sulfurique, l'acide phosphorique ou l'acide acétique.

**16.** Procédé selon l'une des revendications 11 à 15, **caractérisé en ce que** le polyméthylsilsesquioxane est isolé de la solution et lavé et séché à des températures comprises entre environ 80 et 400°C.

**17.** Procédé selon l'une des revendications 11 à 16, **caractérisé en ce que** le polyméthylsiliconate de formule (I) est préparé à partir d'un polyméthylhydrosiloxane de formule générale

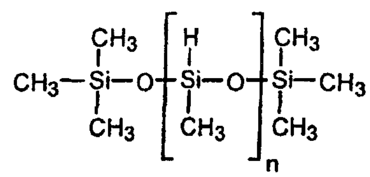

(III)

dans laquelle n est un nombre entier compris entre environ 20 et 100.

**18.** Procédé selon la revendication 17, **caractérisé en ce que** ledit polyméthylsiliconate est préparé par scission de la liaison Si-H à l'aide d'une base aqueuse ou par réaction avec un substrat carbonylé catalysé par un dérivé métallique approprié suivi d'une hydrolyse.

**19.** Polyméthylsilsesquioxane poreux sous forme de poudre, susceptible d'être obtenu par le procédé selon l'une des revendications 11 à 18.

**20.** Utilisation du polyméthylsilsesquioxane selon l'une des revendications 1 à 10 et 19 en tant que support pour l'immobilisation d'enzymes ou pour la préparation d'un tel support.

**21.** Utilisation selon la revendication 20, **caractérisée en ce que** ledit support est préparé à partir dudit polyméthylsilsesquioxane par réaction avec un organosiloxane.

**22.** Utilisation selon la revendication 20 ou 21, **caractérisé en ce que** l'enzyme est une lipase, une peroxidase, une hydrogénase, une lyase, une protéase ou une isomérase.

**23.** Utilisation selon la revendication 20 ou 21, **caractérisé en ce que** ledit enzyme est une lipase capable d'effectuer

la résolution optique d'un mélange d'énantiomères d'un ester ou d'un alcool.

**24.** Utilisation du polyméthylsilsesquioxane selon l'une des revendications 1 à 10 et 19 en tant que support pour des métaux de transition ou des dérivés de ceux-ci.

**25.** Utilisation du polyméthylsilsesquioxane selon l'une des revendications 1 à 10 et 19 en tant qu'adsorbant pour l'extraction de molécules organiques.

**26.** Utilisation du polyméthylsilsesquioxane selon l'une des revendications 1 à 10 et 19 dans l'épuration des gaz et des eaux ou dans la séparation des molécules par chromatographie.

**27.** Utilisation du polyméthylsilsesquioxane selon l'une des revendications 1 à 10 et 19 en tant qu'adsorbant pour des composés odorants.

**28.** Utilisation selon la revendication 27, **caractérisée en ce que** le polyméthylsilsesquioxane contenant des composés odorants est utilisé dans un désodorisant d'air ambiant, dans un savon, dans un shampoing ou autre produit de soins ou d'hygiène capillaire.

**29.** Utilisation selon la revendication 27, **caractérisée en ce que** le polyméthylsilsesquioxane est utilisé dans un savon non parfumé pour adsorber les mauvaises odeurs diffusées par ledit savon.

**30.** Utilisation du polyméthylsilsesquioxane selon l'une des revendications 1 à 10 et 19, en tant que support d'un parfum, pour la préparation de savons, gels de bain ou douche, shampoings, après-shampoings, laques, compositions cosmétiques, détergents ou adoucissants.

**31.** Composition parfumante ou parfumée, **caractérisée en ce qu'**elle contient un polyméthylsesquioxane selon la revendication 9.

**32.** Composition selon la revendication 31, sous forme d'un savon, d'un shampoing ou autre produit de soins ou d'hygiène capillaire.

**Claims**

**1.** A porous polymethylsilsesquioxane in powdered form containing the units

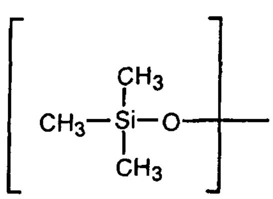

and

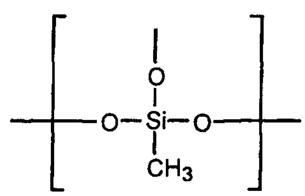

Chain ends

Units of the material

**characterised by** a specific surface area comprised between approximately 50 and 500 $m^2$/g, a pore radius smaller than 1 nm for 90% of the pores, and insolubility in water and organic solvents.

**2.** A polymethylsilsesquioxane according to claim 1, **characterised by** a pore volume comprised between approximately 0. and 0.8 $cm^3$/g.

**3.** A polymethylsilsesquioxane according to claim 1 or 2, **characterised by** a monolayer volume comprised between approximately 10 and 60 $cm^3$/g.

**4.** A polymethylsilsesquioxane according to any one of claims 1 to 3, **characterised by** two adsorption bands around 10° and 22° obtained by X-ray diffraction.

**5.** A polymethylsilsesquioxane according to any one of claims 1 to 4, **characterised by** an average particle size comprised between 1 and 200 µm.

**6.** A polymethylsilsesquioxane according to any one of claims 1 to 5, **characterised by** a thermal stability up to approximately 400°C and a decomposition temperature of approximately 500°C, as determined by differential calorimetry and thermogravimetry.

**7.** A polymethylsilsesquioxane according to any one of claims 1 to 6, **characterised by** a powder density comprised between approximately 0.04 and 0.8 g/$cm^3$.

**8.** A polymethylsilsesquioxane according to any one of claims 1 to 7, **characterised in that** it is a conglomerate of porous nanoparticles with a particle size comprised between approximately 1 and 200 µm.

**9.** A polymethylsilsesquioxane according to any one of claims 1 to 8, **characterised in that** it also contains the units

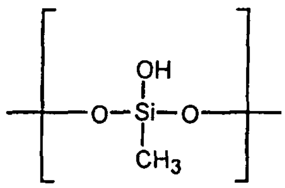

Non-polymerised
silanols

**10.** A polymethylsilsesquioxane according to any one of claims 1 to 9, **characterised by** a specific surface area comprised between approximately 75 and 375 $m^2$/g, a pore volume comprised between approximately 0.2 and 0.7 $cm^3$/g, a monolayer volume comprised between approximately 15 and 50 $cm^3$/g and a pore radius smaller than 0.9 nm for 90% of the pores.

**11.** A process for the preparation of a porous polymethyl-silsesquioxane in powdered form, this process comprising the precipitation, by means of an acid, of an aqueous solution containing a polymethylsiliconate of the general formula

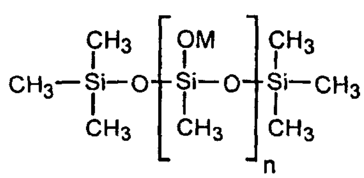

(I)

in which n is a whole number comprised between approximately 20 and 100 and M is an alkaline metal or alkaline-earth metal or $NH_4^+$.

**12.** A process according to claim 11, **characterised in that** the acid is added to the solution until a pH of about at least 11 is reached.

**13.** A process according to claim 11, **characterised in that** the acid is added to the solution until a pH of about at least 7 is reached.

**14.** A process according to any one of claims 11 to 13, **characterised in that** M is sodium or potassium.

**15.** A process according to any one of claims 11 to 14, **characterised in that** the acid used is sulphuric acid, phosphoric acid or acetic acid.

**16.** A process according to any one of claims 11 to 15, **characterised in that** the polymethylsilsesquioxane is isolated from the solution and washed and dried at temperatures between approximately 80 and 400°C.

**17.** A process according to any one of claims 11 to 16, **characterised in that** the polymethylsiliconate of the formula (I) is prepared from a polymethylhydrosiloxane of the general formula

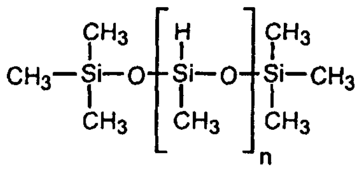

(III)

in which n is a whole number between approximately 20 and 100.

**18.** A process according to claim 17, **characterised in that** the said polymethylsiliconate is prepared by scission of the Si-H bond by means of an aqueous base or by reaction with a carbonyl substrate catalysed by a suitable metal derivative followed by hydrolysis.

**19.** A porous polymethylsilsesquioxane in powdered form obtainable by a process according to any one of claims 11 to 18.

**20.** Use of the polymethylsilsesquioxane according to any one of claims 1 to 10 and 19 as a support for the immobilisation of enzymes or for the preparation of such a support.

**21.** Use according to claim 20, **characterised in that** the said support is prepared from the said polymethylsilsesquioxane by reaction with an organosiloxane.

**22.** Use according to claim 20 or 21, **characterised in that** the enzyme is a lipase, a peroxidase, a hydrogenase, a lyase, a protease or an isomerase.

**23.** Use according to claim 20 or 21, **characterised in that** the said enzyme is a lipase capable of effecting the optical resolution of a mixture of enantiomers of an ester or an alcohol.

**24.** Use of the polymethylsilsesquioxane according to any one of claims 1 to 10 and 19 as a support for transition metals or derivatives thereof.

**25.** Use of the polymethylsilsesquioxane according to any one of claims 1 to 10 and 19 as an adsorbent for the extraction of organic molecules.

**26.** Use of the polymethylsilsesquioxane according to any one of claims 1 to 10 and 19 in the purification of gases and water or in the separation of molecules by chromatography.

**27.** Use of the polymethylsilsesquioxane according to any one of claims 1 to 10 and 19 as an adsorbent for odoriferous compounds.

**28.** Use according to claim 27, **characterised in that** the polymethyl-silsesquioxane containing odoriferous compounds is used in an air freshener, soap, shampoo or other hair-care product.

**29.** Use according to claim 27, **characterised in that** the polymethyl-silsesquioxane is used in a non-perfumed soap in order to adsorb the unpleasant odours diffused by the said soap.

**30.** Use of the polymethylsilsesquioxane according to any one of claims 1 to 10 and 19 as a support for a perfume, for the preparation of soaps, bath or shower gels, shampoos, conditioners, lacquers, cosmetic compositions, detergents or fabric softeners.

**31.** A perfuming or perfumed composition, **characterised in that** it contains a polymethylsilsesquioxane according to

claim 9.

**32.** A composition according to claim 31 in the form of a soap, a shampoo or other hair-care product.

**Patentansprüche**

**1.** Poröses Polymethylsilsesquioxan in Pulverform, welches die Einheiten

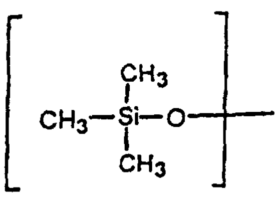

und

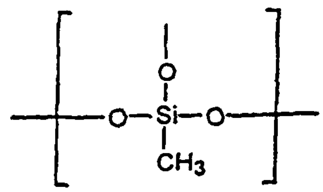

Kettenenden

Bausteine des Materials

enthält, **gekennzeichnet durch** eine spezifische Oberfläche von zwischen ca. 50 und 500 $m^2/g$, einen Porenradius von weniger als 1 nm für 90% der Poren, sowie Unlöslichkeit in Wasser und organischen Lösungsmitteln.

**2.** Polymethylsilsesquioxan nach Anspruch 1, **gekennzeichnet durch** ein Porenvolumen von zwischen ca. 0,1 und 0,8 $cm^3/g$.

**3.** Polymethylsilsesquioxan nach Anspruch 1 oder 2, **gekennzeichnet durch** ein Monoschichtvolumen von zwischen ca. 10 und 60 $cm^3/g$.

**4.** Polymethylsilsesquioxan nach einem der Ansprüche 1 bis 3, **gekennzeichnet durch** zwei mittels Röntgenstrahlenbeugung erhaltene Adsorptionsbanden um 10° und 22°.

**5.** Polymethylsilsesquioxan nach einem der Ansprüche 1 bis 4, **gekennzeichnet durch** eine durchschnittliche Teilchengröße von zwischen ca. 1 und 200 µm.

**6.** Polymethylsilsesquioxan nach einem der Ansprüche 1 bis 5, **gekennzeichnet durch** eine thermische Beständigkeit bis ca. 400°C und eine Zersetzungstemperatur von ca. 500°C gemäß Bestimmung **durch** Differentialkalorimetrie und Thermogravimetrie.

**7.** Polymethylsilsesquioxan nach einem der Ansprüche 1 bis 6, **gekennzeichnet durch** eine Schüttdichte von zwischen ca. 0,04 und 0,8 $g/cm^3$.

**8.** Polymethylsilsesquioxan nach einem der Ansprüche 1 bis 7, **dadurch gekennzeichnet, daß** es sich um ein Konglomerat von porösen Nanopartikeln mit einer Teilchengröße von zwischen ca. 1 und 200 µm handelt.

**9.** Polymethylsilsesquioxan nach einem der Ansprüche 1 bis 8, **dadurch gekennzeichnet, daß** es des weiteren die Einheiten

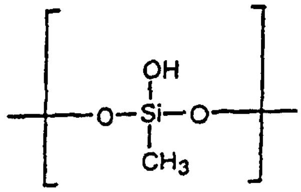

nicht-polymerisierte Silanole

enthält.

10. Polymethylsilsesquioxan nach einem der Ansprüche 1 bis 9, **gekennzeichnet durch** eine spezifische Oberfläche von zwischen ca. 75 und 375 $m^2/g$, ein Porenvolumen von zwischen ca. 0,2 und 0,7 $cm^3/g$, ein Monoschichtvolumen von zwischen ca. 15 und 50 $cm^3/g$, und einen Porenradius von weniger als 0,9 nm für 90% der Poren.

11. Verfahren zur Herstellung eines porösen Polymethylsilsesquioxans in Pulverform, wobei das Verfahren die Präzipitation einer wäßrigen Lösung mit Hilfe einer Säure umfaßt, wobei die wäßrige Lösung ein Polymethylsiliconat der allgemeinen Formel

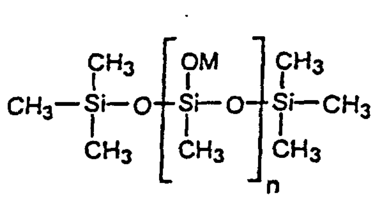 (I)

enthält, in der n eine zwischen ca. 20 und 100 liegende ganze Zahl und M ein Alkali- oder Erdalkalimetall oder $NH_4^+$ ist.

12. Verfahren nach Anspruch 11, **dadurch gekennzeichnet, daß** die Säure der Lösung bis zum Erreichen eines pH-Werts von mindestens ca. 11 zugegeben wird.

13. Verfahren nach Anspruch 11, **dadurch gekennzeichnet, daß** die Säure der Lösung bis zum Erreichen eines pH-Werts von mindestens ca. 7 zugegeben wird.

14. Verfahren nach einem der Ansprüche 11 bis 13, **dadurch gekennzeichnet, daß** M Natrium oder Kalium ist.

15. Verfahren nach einem der Ansprüche 11 bis 14, **dadurch gekennzeichnet, daß** die verwendete Säure Schwefel-, Phosphor- oder Essigsäure ist.

16. Verfahren nach einem der Ansprüche 11 bis 15, **dadurch gekennzeichnet, daß** das Polymethylsilsesquioxan von der Lösung abgetrennt und gewaschen und bei Temperaturen zwischen ca. 80 und 400°C getrocknet wird.

17. Verfahren nach einem der Ansprüche 11 bis 16, **dadurch gekennzeichnet, daß** das Polymethylsiliconat der Formel (I) ausgehend von einem Polymethylhydrosiloxan der allgemeinen Formel

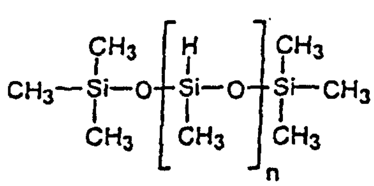 (III)

hergestellt wird, in der n eine zwischen ca. 20 und 100 liegende ganze Zahl ist.

**18.** Verfahren nach Anspruch 17, **dadurch gekennzeichnet, daß** das Polymethylsiliconat durch Spalten der Si-H-Bindung mit Hilfe einer wäßrigen Base oder mittels Umsetzung mit einem durch ein geeignetes Metallderivat katalysierten Carbonylsubstrat, gefolgt von einer Hydrolyse, hergestellt wird.

**19.** Poröses Polymethylsilsesquioxan in Pulverform, welches mittels des Verfahrens nach einem der Ansprüche 11 bis 18 herstellbar ist.

**20.** Verwendung von Polymethylsilsesquioxan nach einem der Ansprüche 1 bis 10 und 19 als Träger für die Fixierung von Enzymen bzw. für die Herstellung eines solchen Trägers.

**21.** Verwendung nach Anspruch 20, **dadurch gekennzeichnet, daß** der Träger ausgehend von dem Polymethylsilsesquioxan durch Umsetzung mit einem Organosiloxan hergestellt wird.

**22.** Verwendung nach Anspruch 20 oder 21, **dadurch gekennzeichnet, daß** das Enzym eine Lipase, eine Peroxidase, eine Hydrogenase, eine Lyase, eine Protease oder eine Isomerase ist.

**23.** Verwendung nach Anspruch 20 oder 21, **dadurch gekennzeichnet, daß** das Enzym eine Lipase ist, die in der Lage ist, die Antipodentrennung einer Mischung von Enantiomeren eines Esters oder eines Alkohols zu bewirken.

**24.** Verwendung von Polymethylsilsesquioxan nach einem der Ansprüche 1 bis 10 und 19 als Träger für Übergangsmetalle oder deren Derivate.

**25.** Verwendung von Polymethylsilsesquioxan nach einem der Ansprüche 1 bis 10 und 19 als Adsorptionsmittel für die Extrahierung von organischen Molekülen.

**26.** Verwendung von Polymethylsilsesquioxan nach einem der Ansprüche 1 bis 10 und 19 bei der Gasreinigung und Wasseraufbereitung oder bei der Trennung von Molekülen mittels Chromatographie.

**27.** Verwendung von Polymethylsilsesquioxan nach einem der Ansprüche 1 bis 10 und 19 als Adsorptionsmittel für Riechstoffverbindungen.

**28.** Verwendung nach Anspruch 27, **dadurch gekennzeichnet, daß** das Riechstoffverbindungen enthaltende Polymethylsilsesquioxan in einem Raumluftdeodorant, einer Seife, einem Shampoo oder einem anderen Haarkosmetik- bzw. -pflegemittel verwendet wird.

**29.** Verwendung nach Anspruch 27, **dadurch gekennzeichnet, daß** das Polymethylsilsesquioxan in einer unparfümierten Seife zum Adsorbieren von durch die Seife abgegebenen schlechten Gerüchen verwendet wird.

**30.** Verwendung von Polymethylsilsesquioxan nach einem der Ansprüche 1 bis 10 und 19 als Träger für ein Parfüm für die Herstellung von Seifen, Bade- oder Duschgels, Shampoos, nach der Haarwäsche anzuwendenden Pflegemitteln, Haarsprays, kosmetischen Zusammensetzungen, Detergenzien oder Weichspülern.

**31.** Beduftungs- oder Riechstoffzusammensetzung, **dadurch gekennzeichnet, daß** sie ein Polymethylsilsesquioxan nach Anspruch 9 enthält.

**32.** Zusammensetzung nach Anspruch 31 in Form einer Seife, eines Shampoos, oder eines anderen Haarpflegeproduktes.

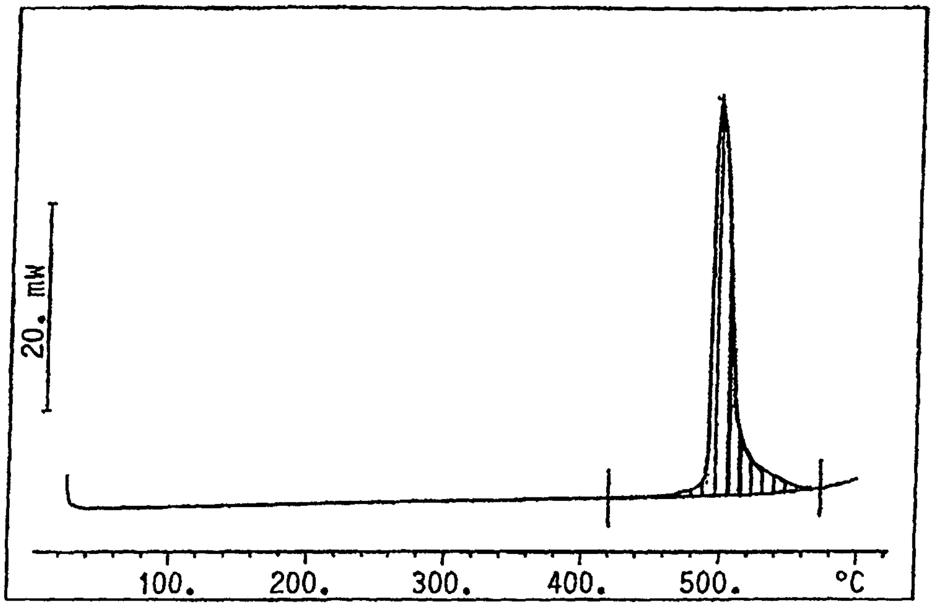


**Fig. 1** Analyse thermique différentielle d'un PMS

**Fig. 2** **Spectres de diffraction X de 2 qualités de PMS poreux**